# EUROPÄISCHE PATENTSCHRIFT

(11) **EP 0 809 637 B1**
(45) Veröffentlichungstag und Bekanntmachung des Hinweises auf die Patenterteilung: **04.06.2003**
(21) Anmeldenummer: 96901336.6
(22) Anmeldetag: 31.01.1996
(51) Int. Cl.: C07D 273/02, C07D 291/02, A01N 43/82, A01N 43/64, A01N 43/88, A01N 43/72, C07D 271/02, C07D 285/00, C07D 249/08, C07D 253/06, C07D 255/02

(54) **FUNGIZIDE AZA-HETEROCYCLOALKENE**
FUNGICIDAL AZA-HETEROCYCLOALKENES
AZA-HETEROCYCLOALCENES ET LEUR UTILISATION COMME FONGICIDES

(30) Priorität: 13.02.1995 DE 19504625; 22.03.1995 DE 19510297
(43) Veröffentlichungstag der Anmeldung: 03.12.1997
(73) Patentinhaber: Bayer CropScience AG, 40789 Monheim (DE)
(72) Erfinder: KRÜGER, Bernd-Wieland, D-51467 Bergisch Gladbach (DE); HEINEMANN, Ulrich, D-42799 Leichlingen (DE); GAYER, Herbert, D-40789 Monheim (DE); ASSMANN, Lutz, D-23701 Eutin (DE); TIEMANN, Ralf, D-51375 Leverkusen (DE); SEITZ, Thomas, D-40764 Langenfeld (DE); HÄNSSLER, Gerd, D-51381 Leverkusen (DE); STENZEL, Klaus, D-40595 Düsseldorf (DE); DUTZMANN, Stefan, D-40721 Hilden (DE)
(86) Internationale Anmeldenummer: EP9600384
(87) Internationale Veröffentlichungsnummer: WO96025406

(56) Entgegenhaltungen:
- EP-A- 0 104 940
- WO-A-94/22844
- WO-A-95/26956
- DE-A- 4 408 005
- DATABASE CROSSFIRE Beilstein Informationssysteme GmbH, Frankfurt DE XP002001737 & HELV. CHIM. ACTA, Bd. 63, Nr. 4, 1980, Seiten 841-59,
- DATABASE CROSSFIRE Beilstein Informationssysteme GmbH, Frankfurt DE XP002001738 & INDIAN J. CHEM. SECT. B, Bd. 29, Nr. 4, 1990, Seiten 315-8,
- DATABASE CROSSFIRE Beilstein Informationssysteme GmbH, Frankfurt DE XP002001739 & J. INDIAN CHEM. SOC., Bd. 6, 1929, Seite 106, 107
- DATABASE CROSSFIRE Beilstein Informationssysteme GmbH, Frankfurt DE XP002001740 & J. INDIAN CHEM. SOC., Bd. 7, 1930, Seite 961, 964
- DATABASE CROSSFIRE Beilstein Informationssysteme GmbH, Frankfurt DE XP002001741 & J. AMER. CHEM. SOC., Bd. 47, 1925, Seite 389
- DATABASE CROSSFIRE Beilstein Informationssysteme GmbH, Frankfurt DE XP002001742 & SYNTHESIS, Bd. 12, 1989, Seiten 923-9,
- DATABASE CROSSFIRE Beilstein Informationssysteme GmbH, Frankfurt DE XP002001743 & J. INDIAN INST. SCI., <A>, Bd. 16, 1933, Seite 11, 17

## Beschreibung

Die Erfindung betrifft neue substituierte Heterocycloalkene, mehrere Verfahren zu ihrer Herstellung und ihre Verwendung als Fungizide sowie neue Zwischenprodukte und mehrere Verfahren zu deren Herstellung.

Es ist bekannt, dass bestimmte substituierte heterocyclische Verbindungen fungizide Eigenschaften aufweisen (vgl. z. B. WO-A 9422844). Die Wirkung dieser Verbindungen ist jedoch insbesondere bei niedrigen Aufwandmengen nicht in allen Anwendungsgebieten völlig zufriedenstellend.

In WO 95/26956 (= EP-A1-754 684) werden Oxim-Derivate offenbart. Tilley et al (Helv. Chim. Acta, 63(4), (1980), 841-859) beschreiben 2,4-Dihydro-3H-1,2,4-triazol-**3-on**-Derivate. Joshua et al. (J. Chem. Sect. B, 29(4), (1990), 315-318) beschreiben 4-Methyl-2,4-dihydro-3H-1,2,4-triazole-**3-thion**-Derivate. Guha et al. (J. Indian Chem. Soc. 7, (1930), 933, 943). Bose et al. (J. Indian Chem. Soc., 7, (1930), 961, 964 offenbaren 1,3,4-Thiadiazol-**2(3H)-on**-Derivate. Guha et al. (J. Amer. Chem. Soc., 47, (1925), 389)offenbaren 1,3,4-Thiadiazole-**2(3H)-thion**-Derivate. Molina et al. (Synthesis, 12, (1989), 923-929), beschreiben ebenfalls 1,3,4-Thiadiazol-**2(3H)-on**-Derivate. Janiiah et al. (J. Indian Inst. Sci., 16, (1993), 11, 17) beschreiben ebenfalls 1,3,4-Thiadiazole-**2(3H)-thion**-Derivate. In WO 94/22844 werden fungizide Oxazolyl- und Oxadiazolyl-Derivate beschrieben. In EP-A-0 104 940 werden 3-Aryl-5,6-dihydro-1,4,2-oxathiazine und ihre Oxide beschrieben. In DE-A- 44 08 005 werden substituierte Azadioxacycloalkene offenbart.

Es wurden nun die neuen substituierten Heterocycloalkene der allgemeinen Formel (I) gefunden, 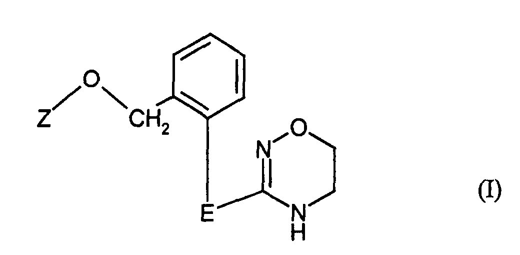 in welcher
- E: für eine der nachstehenden Gruppierungen steht, 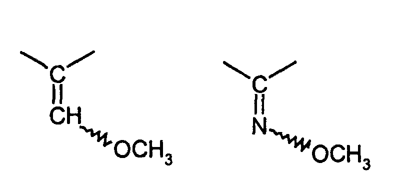
- Z: für jeweils gegebenenfalls einfach bis dreifach, gleich oder verschieden substituiertes Phenyl steht, wobei die möglichen Substituenten vorzugsweise aus der nachstehenden Aufzählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl, Methoxycarbonyl, Ethoxycarbonyl,
jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy oder Ethylendioxy, oder eine Gruppierung 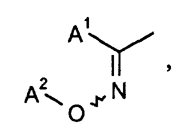 worin
A¹ für Methyl, Ethyl, n- oder i-Propyl, n-, i-, s- oder t-Butyl, Cyclopropyl oder Cyclobutyl und
A² für Methyl, Ethyl, n- oder i-Propyl, n-, i-, s- oder t-Butyl, Allyl, Propargyl, But-2-en-1-yl, 2-Methyl-prop-1-en-3-yl, Cyanmethyl, Methoxymethyl, Ethoxymethyl, Methoxyethyl, Ethoxyethyl, Methylthiomethyl, Ethylthiomethyl, Methylthioethyl, Ethylthioethyl, Dimethylaminomethyl, Dimethylaminoethyl, Methylaminomethyl, Methylaminoethyl oder Benzyl steht.

Weiterhin wurde gefunden, dass man die neuen substituierten Heterocycloalkene der allgemeinen Formel (I) erhält, wenn man
a) Hydroxyverbindungen der allgemeinen Formel (II) 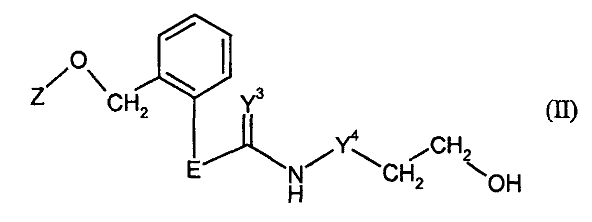 in welcher
   E und Z die oben angegebene Bedeutung haben, wobei
   - Y³: für eine Imino-Gruppierung ("Azamethylen", NH) steht und
   - Y⁴: für Sauerstoff steht,
   mit einem Kondensationsmittel, gegebenenfalls in Gegenwart eines Verdünnungsmittels, umsetzt,
   oder wenn man
b) Stickstoffhaltige Carbonsäurederivate der allgemeinen Formel (III) 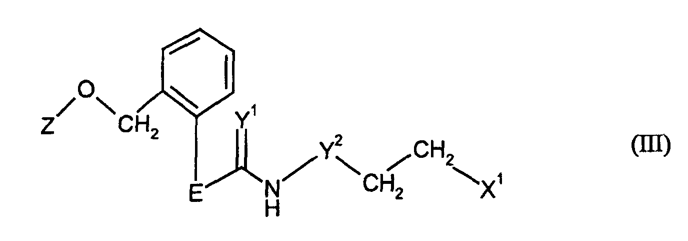
in welcher
- Y¹: für eine Imino-Gruppierung ("Azamethylen", NH) steht,
- Y²: für Sauerstoff steht,
- A, Ar, E, G und Z: die oben angegebene Bedeutung haben und
- X¹: für Halogen, Arylsulfonyl oder Alkylsulfonyl steht,
mit einem Säureakzeptor, gegebenenfalls in Gegenwart eines Verdünnungsmittels, umsetzt.

Schließlich wurde gefunden, dass die neuen substituierten Heterocycloalkene der allgemeinen Formel (I) sehr starke fungizide Wirkung zeigen.

Die erfindungsgemäßen Verbindungen können gegebenenfalls als Mischungen verschiedener möglicher isomerer Formen, insbesondere von Stereoisomeren, wie z. B. E-und Z-Isomeren, gegebenenfalls aber auch von Tautomeren vorliegen. Es werden sowohl die E- als auch die Z-Isomeren, beliebige Mischungen dieser Isomeren, sowie die möglichen tautomeren Formen beansprucht.

In den Definitionen sind die gesättigten oder ungesättigten Kohlenwasserstoffketten, wie Alkyl, Alkandiyl, Alkenyl oder Alkinyl, auch in Verknüpfung mit Heteroatomen, wie in Alkoxy, Alkylthio oder Alkylamino, jeweils geradkettig oder verzweigt.

Halogen steht im Allgemeinen für Fluor, Chlor, Brom oder Iod, vorzugsweise für Fluor, Chlor oder Brom, insbesondere für Fluor oder Chlor.

Die oben aufgeführten Restedefinitionen gelten sowohl für die Endprodukte der Formel (I) als auch entsprechend für die jeweils zur Herstellung benötigten Ausgangsstoffe bzw. Zwischenprodukte.

Beispiele für die erfindungsgemäßen Verbindungen sind in der Tabelle 7 aufgeführt:

### Tabelle 7

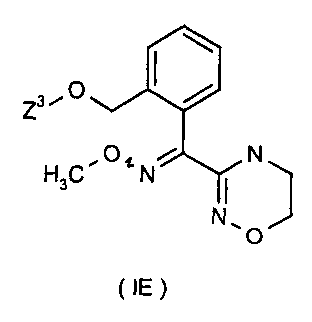

wobei Z³ für die folgenden Substituenten steht:

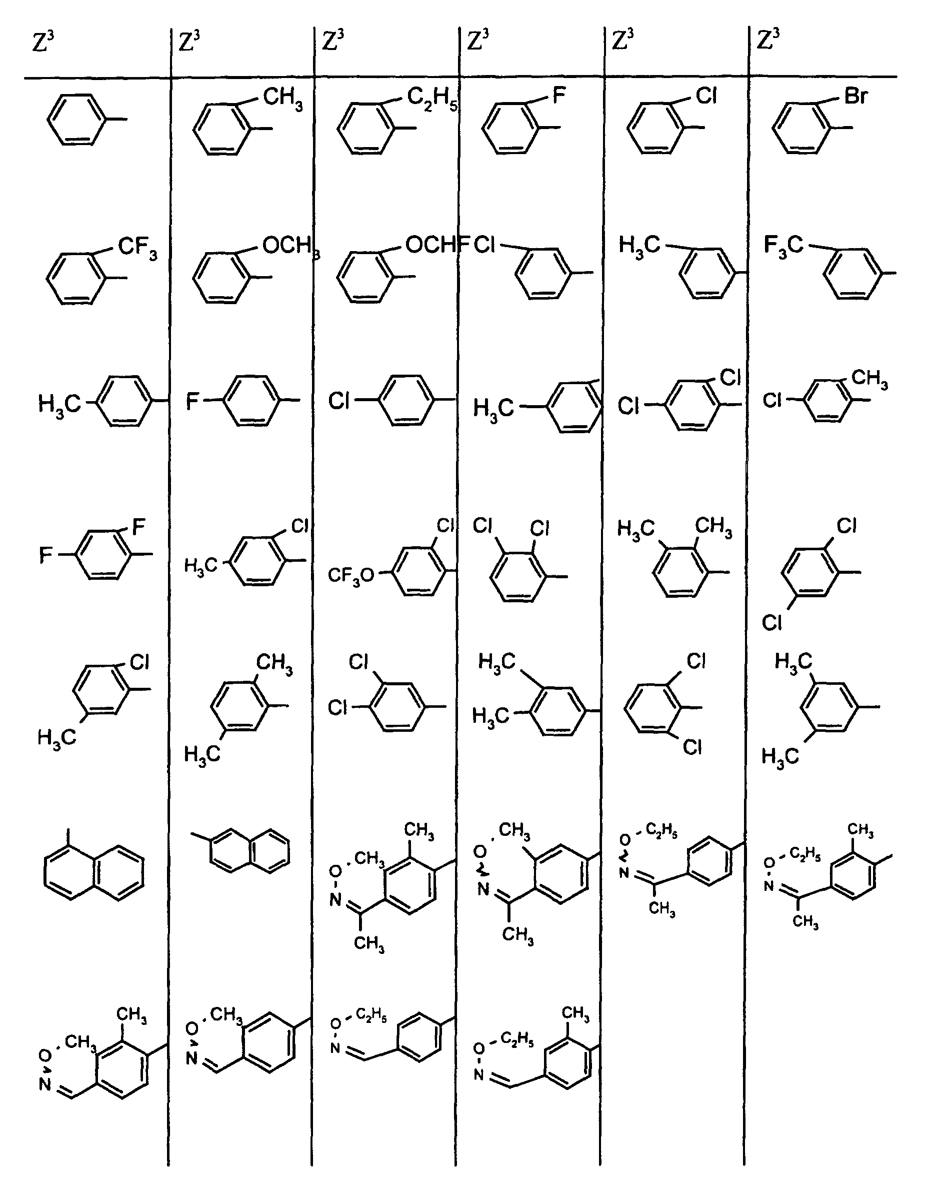

Die zur Durchführung des erfindungsgemäßen Verfahrens a) als Ausgangsstoffe benötigten Hydroxyverbindungen sind durch die Formel (II) allgemein definiert. In dieser Formel (II) haben E, vorzugsweise bzw. insbesondere diejenige Bedeutung, die bereits im Zusammenhang mit der Beschreibung der erfindungsgemäßen Verbindungen der Formel (I) für und Z angegeben wurde. Y³ steht für eine Imino-Gruppierung und Y⁴ steht für eine Imino-Gruppierung.

Die Hydroxyverbindungen der Formel (II) sind noch nicht bekannt. Stehen Y³ und Y⁴ nicht gleichzeitig für Sauerstoff, sind sie Gegenstand der vorliegenden Anmeldung.

Weiterhin wurde gefunden, dass die Hydroxyverbindungen der allgemeinen Formel (II) ebenfalls eine sehr starke fungizide Wirkung zeigen.

Die Hydroxyverbindungen der Formel (II) werden erhalten (Verfahren a-1)), wenn man Carbonsäurederivate der allgemeinen Formel (IV), 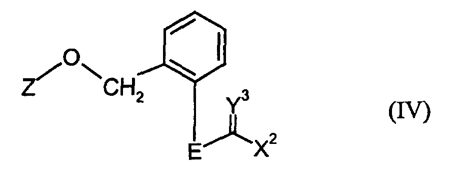 in welcher
- E, Y³ und Z: die oben angegebene Bedeutung haben und
- X²: für Halogen oder Alkoxy steht,
mit einer Hydroxyalkylverbindung der Formel (V), 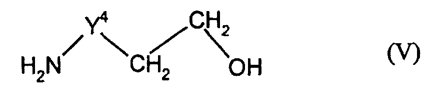 in welcher
- Y⁴: die oben angegebene Bedeutung hat,
gegebenenfalls in Gegenwart eines Verdünnungsmittels, wie beispielsweise Toluol, Pyridin, Dichlormethan oder Tetrahydrofuran und gegebenenfalls in Gegenwart eines Säureakzeptors, wie beispielsweise Triethylamin, Pyridin, Dimethylaminopyridin, Natriumhydroxid oder Kaliumcarbonat, bei Temperaturen von -20 bis 100°C, bevorzugt 0 bis 80°C, umsetzt.

Die zur Durchführung des erfindungsgemäßen Verfahrens a-1) zur Herstellung der Hydroxyverbindungen der allgemeinen Formel (II) als Ausgangsstoffe benötigten Carbonsäurederivate sind durch die Formel (IV) allgemein definiert. In dieser Formel (IV) haben E, Y³ und Z vorzugsweise bzw. insbesondere diejenige Bedeutung, die bereits im Zusammenhang mit der Beschreibung der erfindungsgemäßen Verbindungen der Formel (I), bzw. der Hydroxyverbindungen der Formel (II), als bevorzugt bzw. als insbesondere bevorzugt für E,Y³ und Z angegeben wurde. X² steht für Halogen, bevorzugt Chlor, oder für Alkoxy, bevorzugt Ethoxy oder Methoxy.

Die Carbonsäurederivate der Formel (IV) sind bekannt und/oder können nach an sich bekannten Verfahren hergestellt werden (vgl. EP-A 178826, EP-A 242081, EP-A 382375, EP-A 493711, EP-A 432503, DE-A 3938054, EP-A 528 681).

Die weiterhin zur Durchführung des erfindungsgemäßen Verfahrens a-1) zur Herstellung der Hydroxyverbindungen der allgemeinen Formel (II) als Ausgangsstoffe benötigten Hydroxyalkylverbindungen sind durch die Formel (V) allgemein definiert. In dieser Formel (V) hat Y⁴ vorzugsweise bzw. insbesondere diejenige Bedeutung, die bereits im Zusammenhang mit der Beschreibung der erfindungsgemäßen Verbindungen der Formel (I), bzw. der Hydroxyverbindungen der Formel (II), als bevorzugt bzw. als insbesondere bevorzugt für Y⁴ angegeben wurde.

Die Hydroxyalkylverbindungen der Formel (V) sind bekannt und/oder können nach an sich bekannten Verfahren hergestellt werden (vgl. z. B. J. Chem. Soc., Chem. Com. 1986, 903 oder J. Med. Chem. 1968, 504.

Das erfindungsgemäße Verfahren a) wird gegebenenfalls in Gegenwart eines geeigneten Verdünnungsmittels durchgeführt. Als Verdünnungsmittel zur Durchführung des erfindungsgemäßen Verfahrens a) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; oder Sufone, wie Sulfolan.

Das erfindungsgemäße Verfahren a) wird gegebenenfalls in Gegenwart eines geeigneten Kondensationsmittels durchgeführt. Als solche kommen alle üblichen Reagenzien infrage, die in der Lage sind, von einem Molekül Wasser abzuspalten. Beispielhaft genannt seien Säurehalogenidbildner wie Phosgen, Phosphortribromid, Phosphortrichlorid, Phosphorpentachlorid, Phosphoroxychlorid oder Thionylchlorid; Anhydridbildner wie Chlorameisensäureethylester, Chlorameisensäuremethylester, Chlorameisensäureisopropylester, Chlorameisensäureisobutylester oder Methansulfonylchlorid; Carbodiimide, wie N,N'-Dicyclohexylcarbodiimid (DCC) oder andere übliche Kondensationsmittel, wie Phosphorpentoxid, Polyphosphorsäure, N,N'-Carbonyldiimidazol, 2-Ethoxy-N-ethoxycarbonyl-1,2-dihydrochinolin (EEDQ) oder Triphenylphosphin/Tetrachlorkohlenstoff.

Die Reaktionstemperaturen können bei der Durchführung des erfindungsgemäßen Verfahrens a) in einem größeren Bereich variiert werden. Im Allgemeinen arbeitet man bei Temperaturen zwischen 0°C und +200°C, vorzugsweise bei Temperaturen zwischen 10°C und 140°C.

Zur Durchführung des erfindungsgemäßen Verfahrens a) zur Herstellung der Verbindungen der Formel (I) setzt man pro Mol Hydroxyverbindung der Formel (II) im Allgemeinen 0,1 bis 10 Mol, vorzugsweise 0,1 bis 5 Mol an Schwefelungsreagenz ein.

Die zur Durchführung des erfindungsgemäßen Verfahrens b) als Ausgangsstoffe benötigten stickstoffhaltigen Carbonsäurederivate sind durch die Formel (III) allgemein definiert. In dieser Formel (III) haben E, Y¹, Y² und Z vorzugsweise bzw. insbesondere diejenige Bedeutung, die bereits im Zusammenhang mit der Beschreibung der erfindungsgemäßen Verbindungen der Formel (I) als bevorzugt bzw. als insbesondere bevorzugt für E, Y¹, Y² angegeben wurde. X¹ steht für Halogen, Arylsulfonyl oder Alkylsulfonyl, bevorzugt für Chlor, Methylsulfonyl oder 4-Tolylsulfonyl.

Die stickstoffhaltigen Carbonsäurederivate der Formel (III) sind noch nicht bekannt; sie sind als neue Stoffe Gegenstand der vorliegenden Anmeldung.

Weiterhin wurde gefunden, dass die stickstoffhaltigen Carbonsäurederivate der Formel (III) ebenfalls eine sehr starke fungizide Wirkung zeigen.

Die stickstoffhaltigen Carbonsäurederivate der Formel (III) werden erhalten (Verfahren b-1)), wenn man die bereits weiter oben im Zusammenhang mit der Herstellung der erfindungsgemäßen Verbindungen der Formel (I) nach Verfahren a) beschriebenen Hydroxyverbindungen der Formel (II), mit einem Halogenierungsmittel, wie z.B.

Thionylchlorid, gegebenenfalls in Gegenwart eines Verdünnungsmittels, wie beispielsweise Toluol, Xylol oder Chlorbenzol, gegebenenfalls in Gegenwart eines

Reaktionshilfsmittels, wie beispielsweise Dimethylformamid oder Pyridin, bei Temperaturen von -20 bis 120°C, bevorzugt 0 bis 100°C, oder mit einem Sulfonsäurehalogenid, wie z. b. Methansulfonsäurechlorid oder 4-Toluolsulfonsäurechlorid, gegebenenfalls in Gegenwart eines Verdünnungsmittels, wie beispielsweise Toluol, Pyridin, Dichlormethan oder Tetrahydrofuran und gegebenenfalls in Gegenwart eines Säureakzeptors, wie beispielsweise Triethylamin, Pyridin, Dimethylaminopyridin, Natriumhydroxid oder Kaliumcarbonat, bei Temperaturen von -20 bis 120°C, bevorzugt 0 bis 100°C, umsetzt.

Die weiterhin zur Durchführung des erfindungsgemäßen Verfahrens b-1) zur Herstellung der stickstoffhaltigen Carbonsäurederivate der allgemeinen Formel (II) als Ausgangsstoffe benötigten Halogenierungsmittel, bzw. Sulfonsäurehalogenide sind allgemein bekannte Reagenzien in der organischen Chemie.

Als weiterhin zur Durchführung des erfindungsgemäßen Verfahrens b) benötigte Säureakzeptoren kommen alle üblichen anorganischen oder organischen Basen infrage. Hierzu gehören beispielsweise Erdalkalimetall- oder Alkalimetallhydride, -hydroxide, - amide, -alkoholate, -acetate, -carbonate oder -hydrogencarbonate, wie beispielsweise Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Als Verdünnungsmittel zur Durchführung des erfindungsgemäßen Verfahrens b) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sufone, wie Sulfolan; Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, deren Gemische mit Wasser oder reines Wasser.

Die Reaktionstemperaturen können bei der Durchführung des erfindungsgemäßen Verfahrens b) in einem größeren Bereich variiert werden. Im Allgemeinen arbeitet man bei Temperaturen zwischen -20°C und +130°C, vorzugsweise bei Temperaturen zwischen 0°C und 100°C.

Zur Durchführung des erfindungsgemäßen Verfahrens b) zur Herstellung der Verbindungen der Formel (I) setzt man pro Mol stickstoffhaltigen Carbonsäurederivates der Formel (II) im Allgemeinen 0,8 bis 15 Mol, vorzugsweise 0,8 bis 8 Mol des Säureakzeptors ein.

Die erfindungsgemäßen Verfahren a) und b) werden im Allgemeinen unter Normaldruck durchgeführt. Es ist jedoch auch möglich, unter erhöhtem oder vermindertem Druck - im Allgemeinen zwischen 0,1 bar und 10 bar - zu arbeiten.

Die Reaktionsdurchführung, Aufarbeitung und Isolierung der Reaktionsprodukte erfolgt nach bekannten Verfahren (vgl. auch die Herstellungsbeispiele).

Die erfindungsgemäßen Wirkstoffe weisen eine starke mikrobizide Wirkung auf und werden zur Bekämpfung von unerwünschten Mikroorganismen praktisch eingesetzt. Die Wirkstoffe sind für den Gebrauch als Pflanzenschutzmittel, insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Krankheiten, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubense;
Plasmopara-Arten, wie beispielsweise Plasmopara viticola;
Peronospora-Arten, wie beispielsweise Peronospora pisi oder Peronospora brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielweise Pyrenophora teres oder Pyrenophora graminea (Konidienform: Drechslera, Synonym: Helminthosporium);
Cochliobolus-Arten, wie beispielsweise Cochliobolus sativus (Konidienform: Drechslera, Synonym: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Altemaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen, erlaubt eine Behandlung von oberirdischen Pflanzenteilen, sowie auch eine Behandlung von Pflanz- und Saatgut und des Bodens.

Dabei werden die erfindungsgemäßen Wirkstoffe mit besonders gutem Erfolg zur Bekämpfung von Getreidekrankheiten, wie beispielsweise gegen Erysiphe-Arten oder von Krankheiten im Wein-, Obst- und Gemüseanbau, wie beispielsweise gegen Venturia-, Podospherea- und Sphaerotheca-Arten eingesetzt. Mit gutem Erfolg werden mit den erfindungsgemäßen Wirkstoffen auch Reiskrankheiten, wie beispielsweise Pyricularia-Arten, bekämpft.

Die Wirkstoffe werden in Abhängigkeit von ihren jeweiligen physikalischen und/oder chemischen Eigenschaften gegebenenfalls in übliche Formulierungen übergeführt, wie z. B. Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und -Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, Alkylnaphthaline, chlorierte Aromaten oder chlorierte aliphatische Kohlenwasserstoffe, wie Chlorbenzole, Chlorethylene, oder Methylenchlorid, aliphatsche Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid oder Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen infrage: z.B. natürliche Gesteinsmehle, wie Kaoline, Tonerden, Talkum, Kreide, Quartz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infrage: z.B. nicht ionogene und anionische Emulgatoren, wie Polyoxyethylen-Fettsäureester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen infrage: z.B. Ligninsulfitablaugen und Methylcellulose.

Es werden in den Formulierungen gegebenenfalls Haftmittel wie Carboxymethylcellulose, natürliche und synthetische, pulverige, körnige oder latexförmige Polymere verwendet, wie z. B. Gummiarabicum, Polyvinylalkohol, Polyvinylacetat, sowie natürliche Phospholipide, wie Kephaline und Lecithine und synthetische Phospholipide. Weitere mögliche Additive sind mineralische und vegetabile Öle.

Gegebenenfalls werden Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink zugesetzt.

Die Formulierungen enthalten im Allgemeinenzwischen 0,1 und 95 Gewichtsprozent Wirkstoff, vorzugsweise zwischen 0,5 und 90 %.

Die erfindungsgemäßen Wirkstoffe werden als solche oder in ihren Formulierungen auch in Mischung mit bekannten Fungiziden, Bakteriziden, Akariziden, Nematiziden oder Insektiziden verwendet, um so z.B. das Wirkungsspektrum zu verbreitern oder Resistenzentwicklungen vorzubeugen.

In vielen Fällen werden dabei synergistische Wirkungen beobachtet.

Für die Mischungen kommen beispielsweise infrage:
**Fungizide:**
   2-Aminobutan; 2-Anilino-4-methyl-6-cyclopropyl-pyrimidin; 2',6'-Dibromo-2-methyl-4'-trifluoromethoxy-4'-trifluoro-methyl-1,3-thizole-5-carboxanilid; 2,6-Dichloro-N-(4-trifluoromethylbenzyl)benzamid; (E)-2-Methoxyimino-N-methyl-2-(2-phenoxyphenyl) acetamid; 8-Hydroxyquinolinsulfat; Methyl-(E)-2-{2-[6-(2-cyano-phenoxy)pyrimidin-4-yloxy]phenyl}-3-methoxyacrylat; Methyl-(E)-methoximino [alpha-(o-tolyloxy)-otolyl] acetat; 2-Phenylphenol (OPP), Aldimorph, Ampropylfos, Anilazin, Azaconazol,
   Benalaxyl, Benodanil, Benomyl, Binapacryl, Biphenyl, Bitertanol, Blasticidin-S, Bromuconazole, Bupirimate, Buthiobate,
   Calciumpolysulfid, Captafol, Captan, Carbendazim, Carboxin, Chinomethionat (Quinomethionat), Chloroneb, Chloropicrin, Chlorothalonil, Chlozolinat, Cufraneb,
   Cymoxanil, Cyproconazole, Cyprofuram,
   Dichlorophen, Diclobutrazol, Diclofluanid, Diclomezin, Dicloran, Diethofencarb, Difenoconazol, Dimethirimol, Dimethomorph, Diniconazol, Dinocap, Diphe-nylamin, Dipyrithion, Ditalimfos, Dithianon, Dodin, Drazoxolon,
   Edifenphos, Epoxyconazole, Ethirimol, Etridiazol,
   Fenarimol, Fenbuconazole, Fenfuram, Fenitropan, Fenpiclonil, Fenpropidin, Fenpropimorph, Fentinacetate, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
   Guazatine,
   Hexachlorobenzol, Hexaconazol, Hymexazol,
   Imazalil, Imibenconazol, Iminoctadin, Iprobenfos (IBP), Iprodion, Isoprothiolan,
   Kasugamycin, Kupfer-Zubereitungen, wie: Kupferhydroxid, Kupfernaphthenat, Kupferoxychlorid, Kupfersulfat, Kupferoxid, Oxin-Kupfer and Bordeaux-Mischung,
   Mancopper, Mancozeb, Maneb, Mepanipyrim, Mepronil, Metalaxyl, Metconazol,
   Methasulfocarb, Methfuroxam, Metiram, Metsulfovax, Myclobutanil,
   Nickel dimethyldithiocarbamat, Nitrothal-isopropyl, Nuarimol,
   Ofurace, Oxadixyl, Oxamocarb, Oxycarboxin,
   Pefurazoat, Penconazol, Pencycuron, Phosdiphen, Pimaricin, Piperalin, Polyoxin, Probenazol, Prochloraz, Procymidon, Propamocarb, Propiconazole, Propineb, Pyrazophos, Pyrifenox, Pyrimethanil, Pyroquilon,
   Quintozen (PCNB),
   Schwefel und Schwefel-Zubereitungen,
   Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
   Validamycin A, Vinclozolin,
   Zineb, Ziram
**Bakterizide:**
   Bronopol, Dichlorophen, Nitrapyrin, Nickel Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.
**Insektizide / Akarizide / Nematizide:**
   Abamectin, Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
   Bacillus thuringiensis, Bendiocarb, Benfuracarb, Bensultap, Betacyluthrin, Bifenthrin, BPMC, Brofenprox, Bromophos A, Bufencarb, Buprofezin, Butocarboxin, Butylpyridaben,
   Cadusafos, Carbaryl, Carbofuran, Carbophenothion, Carbosulfan, Cartap, CGA 157 419, CGA 184699, Chloethocarb, Chlorethoxyfos, Chloretoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
   Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
   Edifenphos, Emamectin, Esfenvalerat, Ethiofencarb, Ethion, Ethofenprox, Ethoprophos, Etofenprox, Etrimphos,
   Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
   HCH, Heptenophos, Hexaflumuron, Hexythiazox,
   Imidacloprid, Iprobenfos, Isazophos, Isofenphos, Isoprocarb, Isoxathion, Ivemectin, Lamda-cyhalothrin, Lufenuron,
   Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
   Naled, NC 184, NI 25, Nitenpyram
   Omethoat, Oxamyl, Oxydemethon M, Oxydeprofos,
   Parathion A, Parathion M, Permethrin, Phenthoat, Phorat, Phosalon, Phosmet, Phosphamdon, Phoxim, Pirimicarb, Pirimiphos M, ,Primiphos A, Profenofos, Profenophos, Promecarb, Propaphos, Propoxur, Prothiofos, Prothiophos, Prothoat, Pymetrozin, Pyrachlophos, Pyraclofos, Pyraclophos, Pyradaphenthion, Pyresmethrin, Pyrethrum, Pyridaben, Pyrimidifen, Pyriproxifen,
   Quinalphos,
   RH 5992,
   Salithion, Sebufos, Silafluofen, Sulfotep, Sulprofos,
   Tebufenozid, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
   Vamidothion, XMC, Xylylcarb, YI 5301/5302, Zetamethrin.

Gegebenenfalls werden die erfindungsgemäßen Wirkstoffe auch mit anderen bekannten Wirkstoffen, wie Herbiziden oder auch mit Düngemitteln und Wachstumsregulatoren gemischt.

Die Wirkstoffe werden als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet. Die Anwendung geschieht in üblicher Weise, z.B. durch Gießen, Verspritzen, Versprühen, Verstreuen, Verstäuben, Verschäumen, Bestreichen usw. Gegebenenfalls werden die Wirkstoffe nach dem Ultra-Low-Volume-Verfahren ausgebracht oder die Wirkstoffzubereitung oder der Wirkstoff selbst wird in den Boden injiziert. Gegebenenfalls wird auch das Saatgut der Pflanzen behandelt.

Bei der Behandlung von Pflanzenteilen können die Wirkstoffkonzentrationen in den Anwendungsformen in einem größeren Bereich variiert werden: Sie liegen im Allgemeinen zwischen 1 und 0,0001 Gew.-%, vorzugsweise zwischen 0,5 und 0,001 Gew.-%.

Bei der Saatgutbehandlung werden im Allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

Bei der Saatgutbehandlung werden im Allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

### Herstellungsbeispiele

### Beispiel (I- 1)

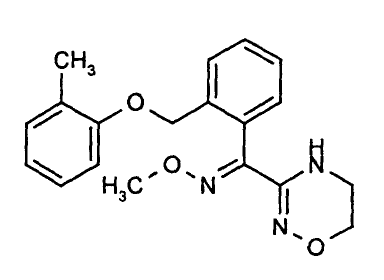

### (Verfahren b))

0,4 g (1,067 mMol) 2-[2-(2-Methylphenoxymethyl)-phenyl]-2-(methoximino)-O-(2-chlorethyl)-acetamidoxim werden in 10 ml N-Methylpyrrolidon gelöst und langsam mit 35 mg Natriumhydrid (1,17 mMol; 80%ig in Paraffin) versetzt. Man rührt 16 h bei 100° C, gießt auf Wasser, extrahiert mehrfach mit Essigsäureethylester, wäscht erneut mit Wasser, trocknet die organische Phase mit Magnesiumsulfat und destilliert das Lösungsmittel im Vakuum ab. Der Rückstand wird dann über Kieselgel chromatographiert (Laufmittel Toluol:Aceton=15:1). Man erhält 0,23 g (64% d. Theorie) 3-{1-[2-(2-Methylphenoxymethyl)-phenyl]-1-(methoximino)-methyl}-5,6-dihydro-4H-1,2,4-oxadiazin. MS: 89, 116, 143, 170, 201, 232, 308, 339 M⁺.

### Beispiel (I-3) (Herstellungsbeispiel außerhalb des stofflichen Umfangs der Erfindung)

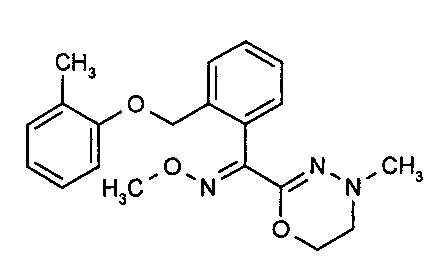

### (Verfahren b))

1,0 g (2,5 mmol) N'-(2-Chlorethyl)-N'-methyl-2-methoximino-2-[2-(2-methylphenoxymethyl)-phenyl]-acethydrazid wird in 20 ml wasserfreiem Toluol vorgelegt und mit 0,35 g (3,1 mmol) Kalium tert-butylat versetzt. Die Mischung wird 15 Minuten unter Rückfluß zum Sieden erhitzt und nach dem Abkühlen nacheinander mit Wasser, verdünnter Salzsäure und wieder mit Wasser gewaschen. Die organische Phase wird über Natriumsulfat getrocknet und im Vakuum eingeengt. Der Rückstand wird mit Petrolether/Essigester (4:1) an Kieselgel chromatografiert. Man erhält 0,39 g (45 % der Theorie 2-{1-Methoximino-1 -[2-(2-methylphenoxymethyl)-phenyl]-methyl} -4-methyl-5.6-dihydro-4H-1.3.4-oxadiazin. logP = 3,54

Analog den Beispielen (I-1) und (I-3), sowie entsprechend der allgemeinen Beschreibung der erfindungsgemäßen Herstellungsverfahren, erhält man auch die in der nachstehenden Tabelle 9 aufgeführten erfindungsgemäßen Verbindungen der Formel (I):

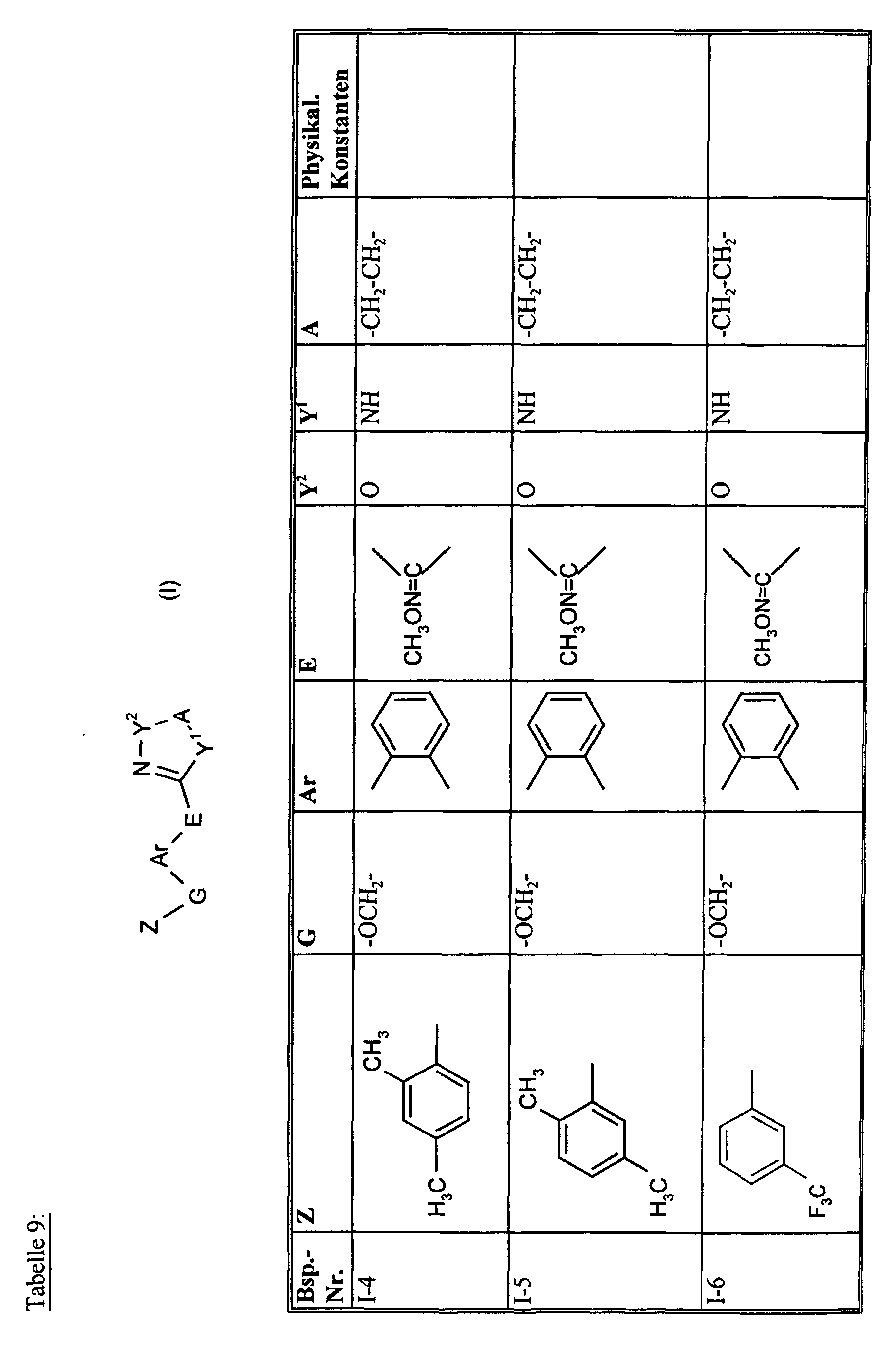

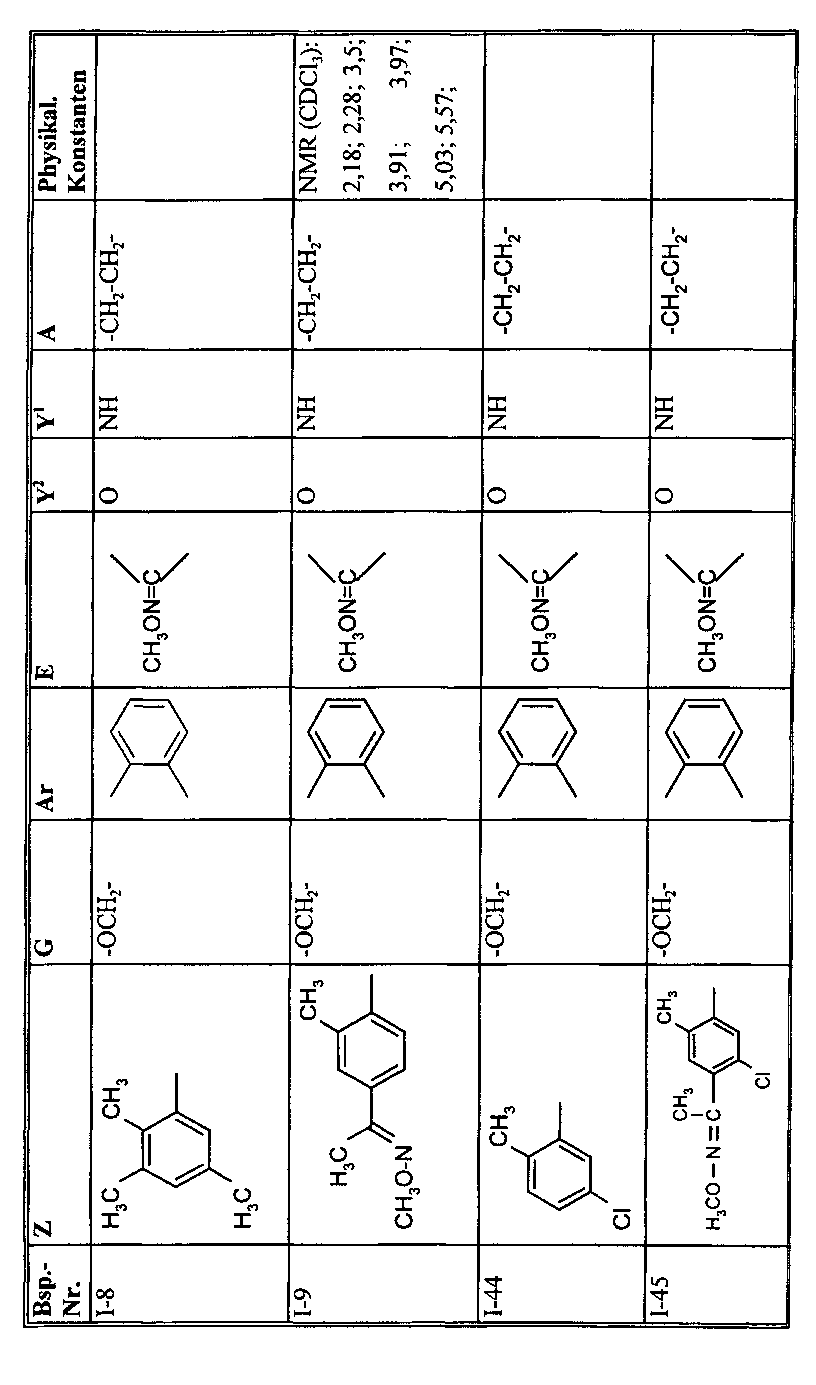

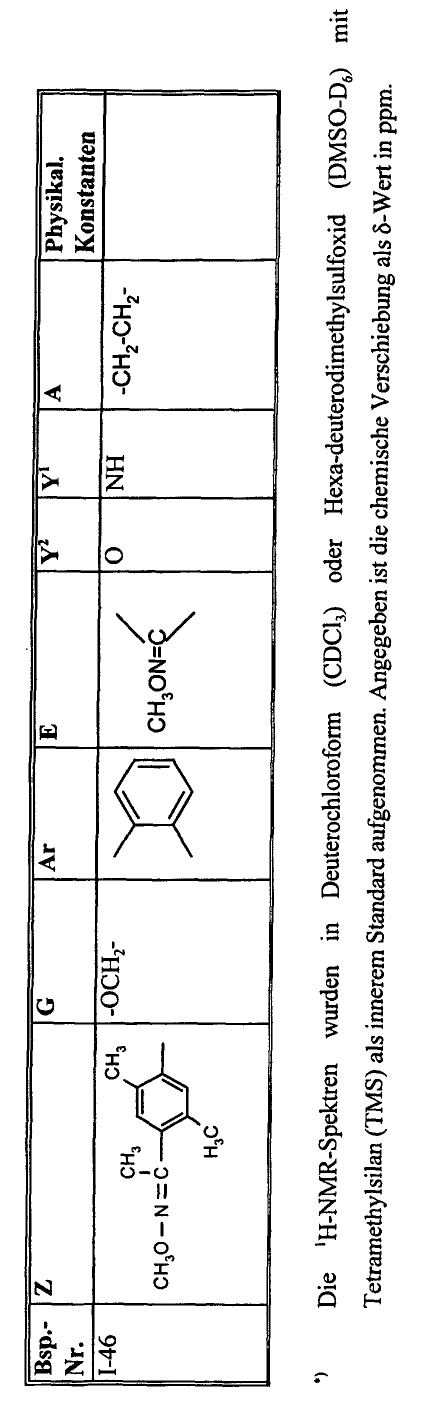

### Herstellung des Ausgangsproduktes Beispiel (II-2)

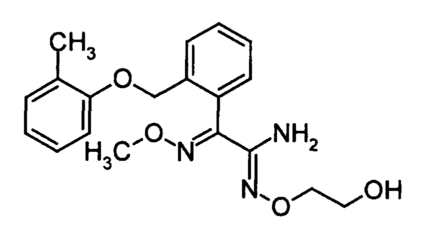

### (Verfahren a-1)

0,5 g (1,6 mMol) 2-[2-(2-Methylphenoxymethyl)-phenyl]-2-(methoximino)-acetimidsäureethylester, 0,25 g (3,21 mMol) 2-Hydroxy-ethyl-hydroxylamin und 10 mg (0,19 mMol) Ammoniumchlorid werden in 5 ml Ethanol suspendiert und 16 Stunden bei 40°C erhitzt. Anschließend gießt man den Reaktionsansatz auf 100 ml Wasser, säuert mit 1N Salzsäure auf pH 2 bis 3 an, extrahiert dreimal mit je 100 ml Essigsäureethylester, vereinigt die organischen Phasen und trocknet über Magnesiumsulfat. Nach Abdestillieren des Essigsäureethylesters bei vermindertem Druck wird der Rückstand über Kieselgel chromatographiert (Laufmittel Cyclohexan:Essigsäureethylester=3:1). Man erhält 0,43 g (69% d. Theorie) 2-[2-(2-Methylphenoxymethyl)-phenyl]-2-(methoximino)-O-(2-hydroxyethylethyl)-acetamidoxim. ¹H NMR: (CDCl₃) 1,58, 2,27, 2,77, 3,73, 3,93, 4,0, 5,0, 5,1, 7,1-7,6 ppm.

### Herstellung des Ausgangsproduktes Beispiel (II-3)

### (Herstellungsbeispiel außerhalb des stofflichen Umfangs der Erfindung)

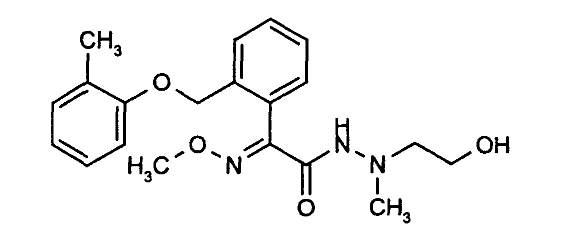

### (Verfahren a-1)

Zu einer Mischung von 0,23 g (3 mmol) 2-(N-Methyl-hydrazino)-ethanol und 0,42 ml (3 mmol) Triethylamin in 30 ml Dichlormethan tropft man innerhalb von 20 Minuten bei 20 °C 0,95 g (3 mmol) 2-Methoximino-2-[2-(2-methylphenoxymethyl)-phenyl]-essigsäurechlorid und rührt 18 Stunden bei 20 °C. Die Mischung wird auf Wasser gegeben und zuerst mit Natriumhydrogencarbonatlösung und dann mit Wasser gewaschen. Die organische Phase wird über Natriumsulfat getrocknet, filtriert und wieder eingeengt. Der Rückstand wird mit Petrolether/Essigester (5:1) an Kieselgel chromatografiert.

Man erhält 0,3 g (27 % der Theorie) N'-(2-Hydroxyethyl)-N'-methyl-2-methoximino-2-[2-(2-methylphenoxymethyl)-phenyl]-acethydrazid vom Schmelzpunkt 68-69°C.

Analog den Beispielen (II-1) bis (II-3), sowie entsprechend der allgemeinen Beschreibung der erfindungsgemäßen Herstellungsverfahren, erhält man auch die in der nachstehenden Tabelle 10 aufgeführten erfindungsgemäßen Verbindungen der Formel (II):

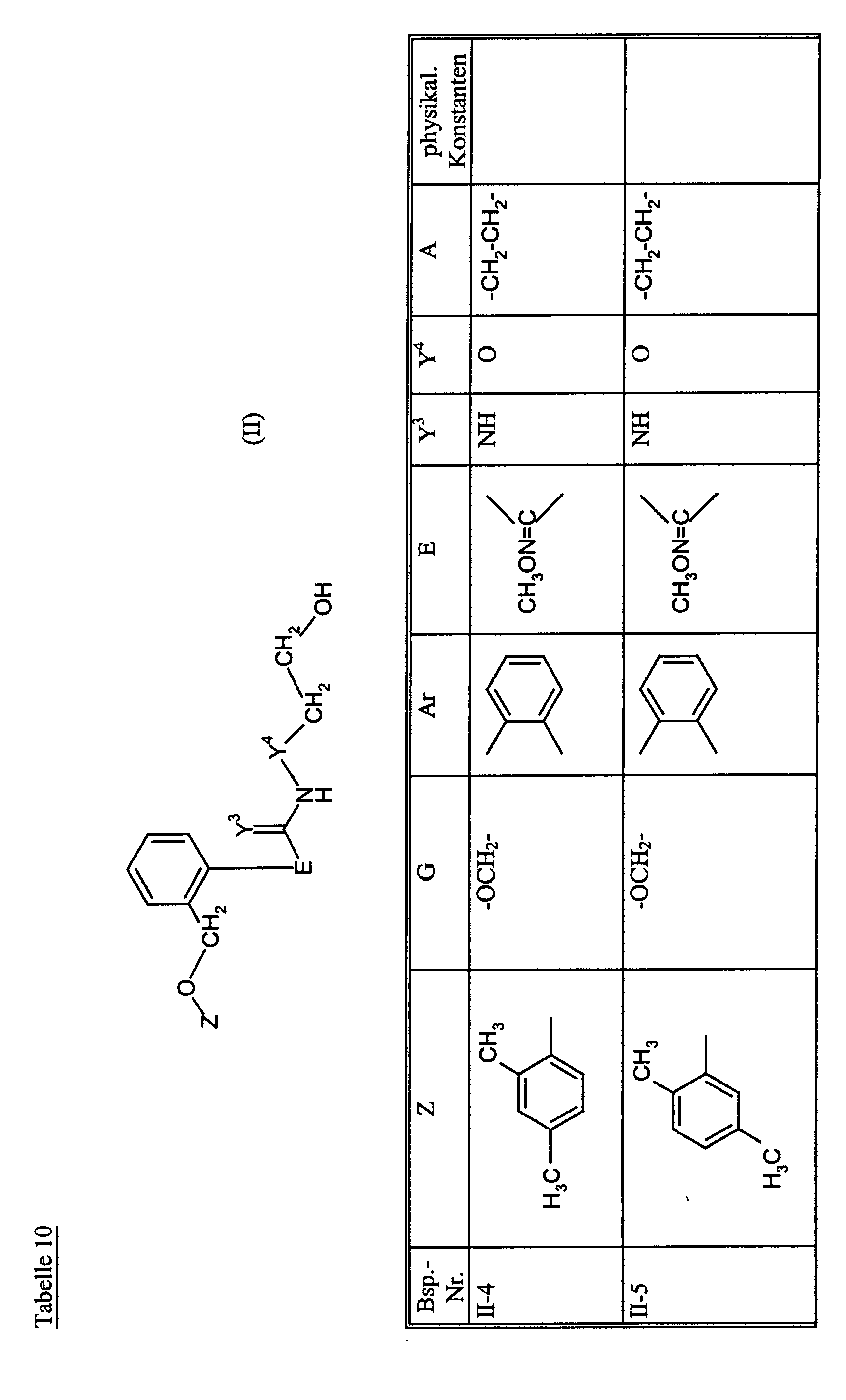

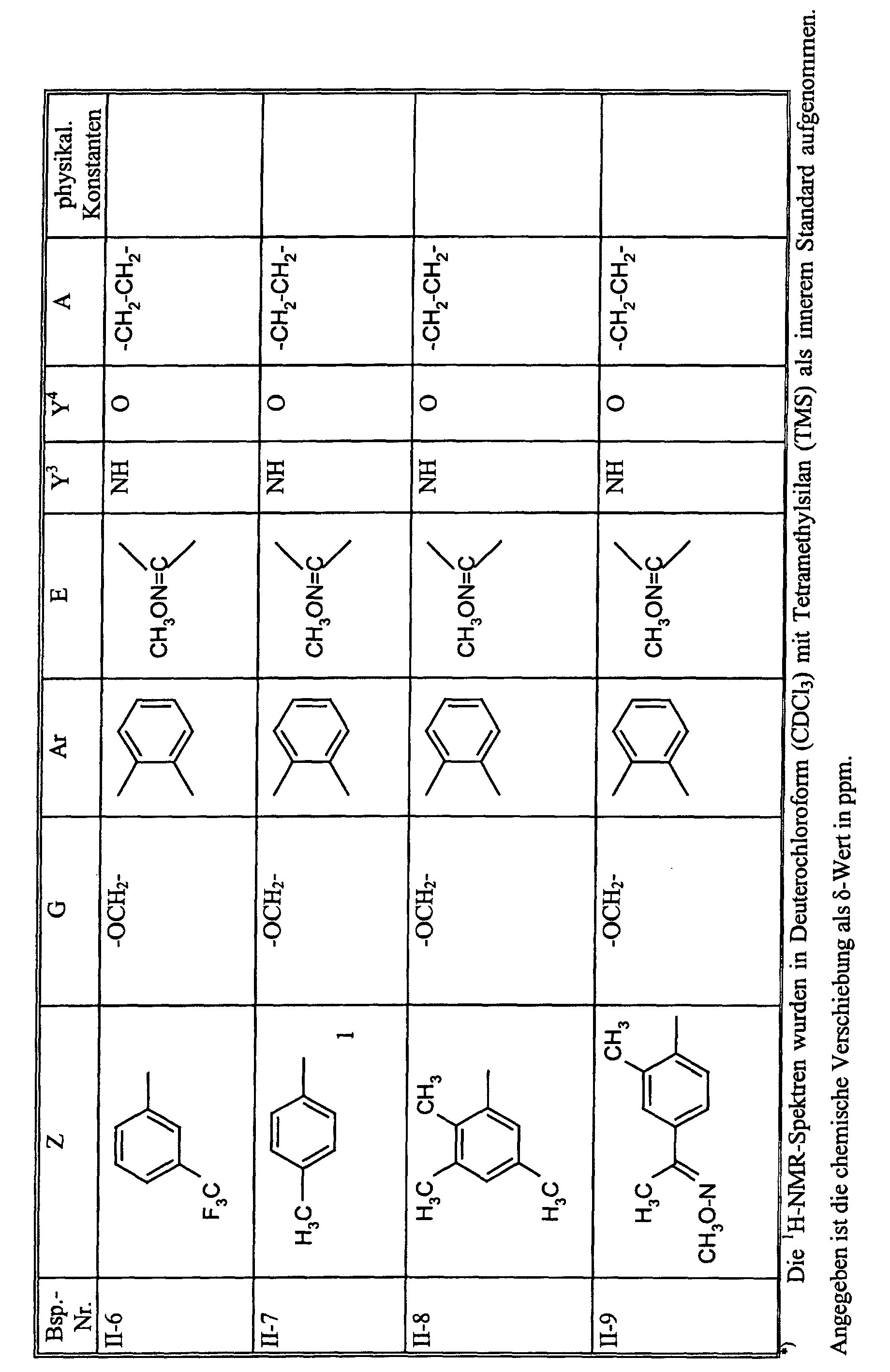

### Herstellung der Ausgangsproduktes, Beispiel (III-1):

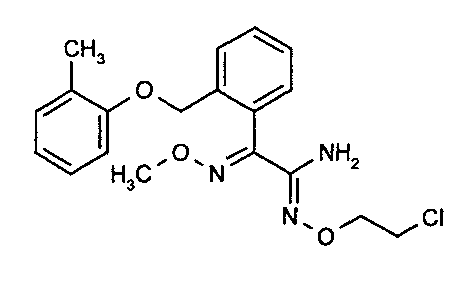

### (Verfahren b-1)

0,5 g (1,29 mMol) 2-[2-(2-Methylphenoxymethyl)-phenyl]-2-(methoximino)-O-(2-hydroxyethyl)-acetamidoxim werden in 5 ml Chloroform gelöst und langsam 1,5 g (12,94 mMol) Thionylchlorid zugetropft. Man rührt 16 h bei 20°C, gießt dann die Reaktionsmischung auf Wasser, extrahiert mit Essigsäureethylester, wäscht erneut mit Wasser, trocknet die organische Phase mit Natriumsulfat und destilliert das Lösungsmittel im Wasserstrahlvakuum ab. Man erhält 0,45 g (94 % d. Theorie) 2-[2-(2-Methylphenoxymethyl)-phenyl]-2-(methoximino)-O-(2-chlorethyl)-acetamidoxim.
MS: 116, 158, 188, 237, 268, 344, 375 M⁺.

### Herstellung des Ausgangsproduktes, Beispiel (III-2)

### (Herstellungsbeispiele außerhalb des stofflichen Umfangs der Erfindung)

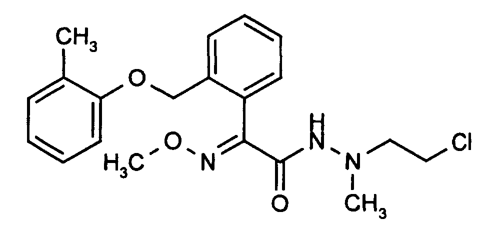

### (Verfahren b-1)

Zu einer Lösung von 1,0 (2,7 mmol) N'-(2-Hydroxyethyl)-N'-methyl-2-methoximino-2-[2-(2-methylphenoxymethyl)-phenyl]-acethydrazid in 20 ml Dichlormethan werden 0,4 ml (5,4 mmol) Thienylchlorid zugetropft. Man lässt die Mischung 4 Stunden bei 20°C rühren und destilliert das Lösungsmittel ab. Der Rückstand wird in Dichlormethan gelöst, nacheinander mit Wasser, Natriumhydrogencarbonatlösung und wieder mit Wasser gewaschen. Die organische Phase wird über Natriumsulfat getrocknet und im Vakuum eingeengt. Man erhält 1,1 g (100 % der Theorie) N'-(2-Chlorethyl)-N'methyl-2-methoximino-2-[2-(2-methylphenoxymethyl)-phenyl]-acethydrazid als Öl, das roh weiter eingesetzt wird.
¹H-NMR (CDCl₃/TMS): δ = 4,08 (s, 3H) ppm

Analog den Beispielen (III-1) und (III-2), sowie entsprechend der allgemeinen Beschreibung der erfindungsgemäßen Herstellungsverfahren, erhält man auch die in der nachstehenden Tabelle 11 aufgeführten erfindungsgemäßen Verbindungen der Formel (III):

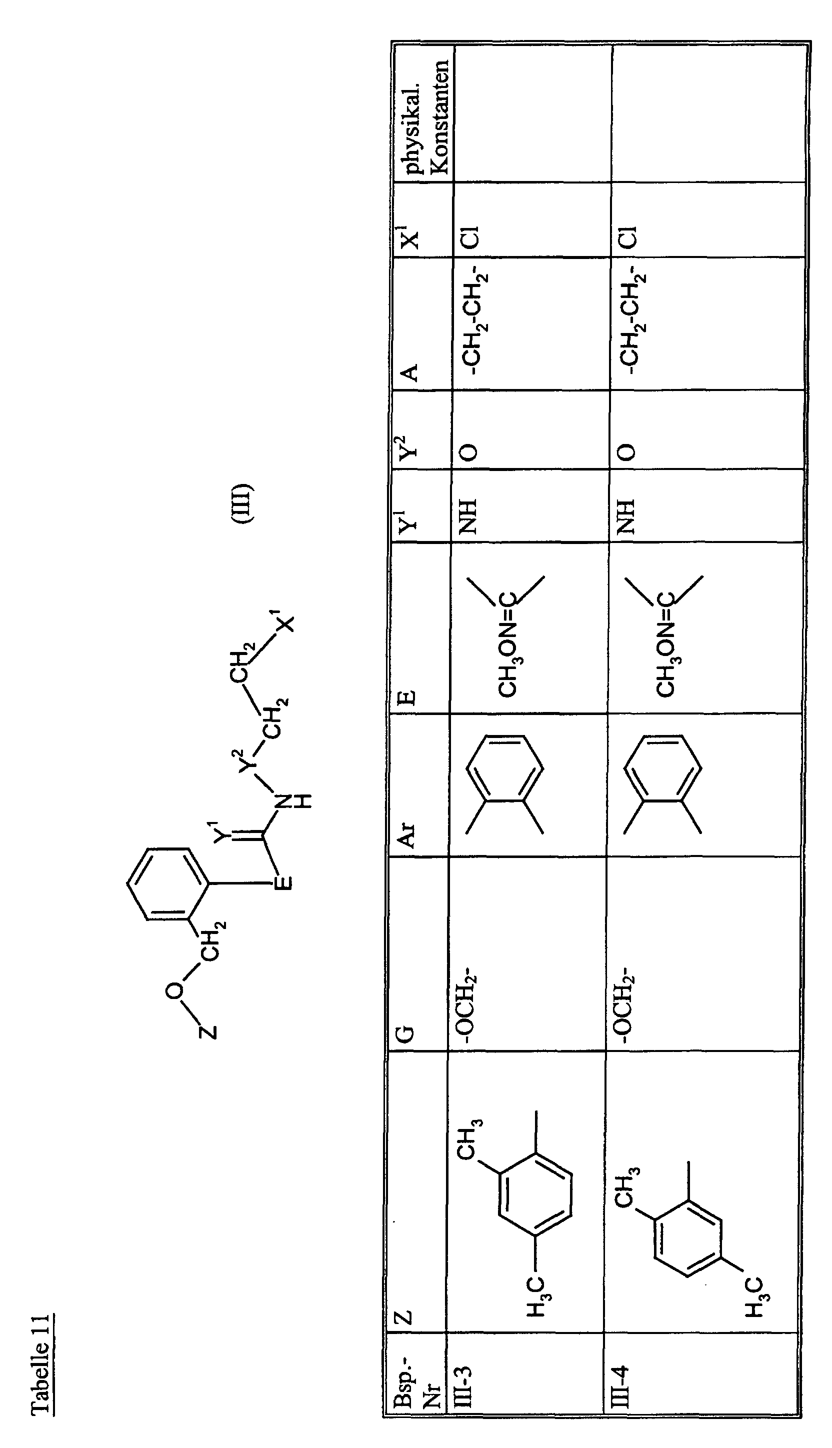

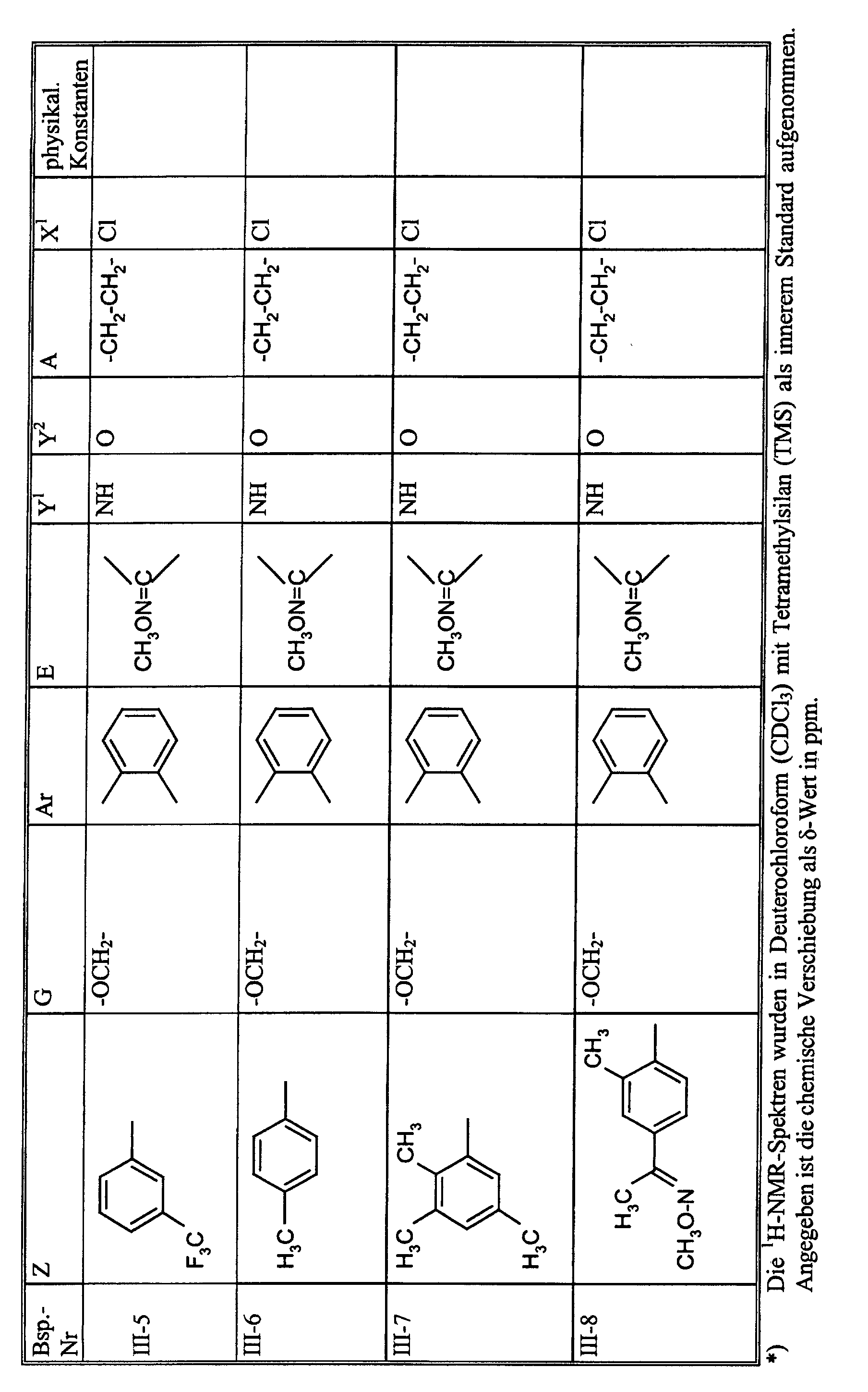

### Beispiel A

| **Venturia-Test (Apfel)** / protektiv | |
|---|---|
| Lösungsmittel | 12,5 Gewichtsteile Aceton |
| Emulgator | 0,3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers Venturia inaequalis inokuliert und verbleiben dann 1 Tag bei 20°C und 100 % relativer Luftfeuchtigkeit in einer Inkubationskabine.

Die Pflanzen werden dann im Gewächshaus bei 20°C und einer relativen Luftfeuchtigkeit von ca. 70 % aufgestellt.

12 Tage nach der Inokulation erfolgt die Auswertung.

Bei diesem Test zeigen z.B. die folgenden Verbindungen (I-1) und (I-2) (Beispiel außerhalb der Erfindung) bei einer Wirkstoffkonzentration von 500 ppm einen Wirkungsgrad von 100 %.

### Beispiel B

| **Erysiphe-Test (Gerste)** / protektiv | |
|---|---|
| Lösungsmittel | 12,5 Gewichtsteile N-Methyl-pyrrolidon |
| Emulgator | 0,3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

Bei diesem Test zeigen z.B. die folgenden Verbindungen (I-1) und (I-2) (Beispiel außerhalb der Erfindung) bei einer Wirkstoffkonzentration von 500 ppm einen Wirkungsgrad von 90 %.

### Beispiel C

| **Podosphaera-Test (Apfel)** / protektiv | |
|---|---|
| Lösungsmittel | 4,7 Gewichtsteile Aceton |
| Emulgator | 0,3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung besprüht. Nach Antrocknen des Spritzbelages werden die Pflanzen durch Bestäuben mit Konidien des Apfelmehltauerregers Podosphaera leucotricha inokuliert.

Die Pflanzen werden dann im Gewächshaus bei 23°C und einer relativen Luftfeuchtigkeit von ca. 70 % aufgestellt.

10 Tage nach der Inokulation erfolgt die Auswertung.

Bei diesem Test zeigt z.B. die folgende Verbindung (I-2) (Beispiel außerhalb der Erfindung) bei einer Wirkstoffkonzentration von 100 ppm einen Wirkungsgrad von 90 %.

### Beispiel D

| **Sphaerotheca-Test (Gurke)** / protektiv | |
|---|---|
| Lösungsmittel | 4,7 Gewichtsteile Aceton |
| Emulgator | 0,3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung besprüht. Nach Antrocknen des Spritzbelages werden die Pflanzen mit Konidien des Pilzes Sphaerotheca fuliginea bestäubt.

Die Pflanzen werden anschließend bei 23 bis 24°C und bei einer relativen Luftfeuchtigkeit von ca. 75 % im Gewächshaus aufgestellt.

10 Tage nach der Inokulation erfolgt die Auswertung.

Bei diesem Test zeigt z.B. die Verbindung (I-2) (Beispiel außerhalb der Erfindung) bei einer Wirkstoffkonzentration von 100 ppm einen Wirkungsgrad von 96 %.

## Patentansprüche

1. Verbindungen der allgemeinen Formel (I), 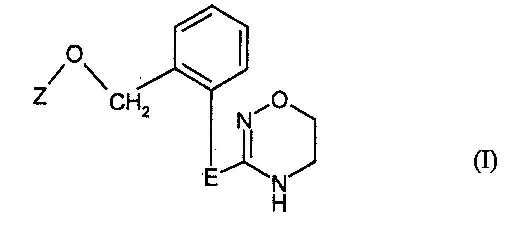 in welcher
E für eine der nachstehenden Gruppierungen steht, 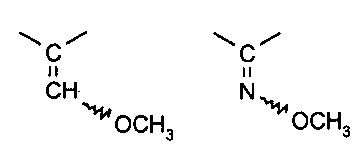 und
Z für jeweils gegebenenfalls einfach bis dreifach, gleich oder verschieden substituiertes Phenyl steht, wobei die möglichen Substituenten vorzugsweise aus der nachstehenden Aufzählung ausgewählt sind:
Fluor, Chlor, Brom, Cyano, Methyl, Ethyl, n- oder i-Propyl, n-, i-, s-oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl, Methoxycarbonyl, Ethoxycarbonyl,
jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy oder Ethylendioxy,
oder eine Gruppierung 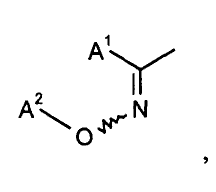 worin
A¹ für Methyl, Ethyl, n- oder i-Propyl, n-, i-, s- oder t-Butyl, Cyclopropyl oder Cyclobutyl und
A² für Methyl, Ethyl, n- oder i-Propyl, n-, i-, s- oder t-Butyl, Allyl, Propargyl, But-2-en-1-yl, 2-Methyl-prop-1-en-3-yl, Cyanmethyl, Methoxymethyl, Ethoxymethyl, Methoxyethyl, Ethoxyethyl, Methylthiomethyl, Ethylthiomethyl, Methylthioethyl, Ethylthioethyl, Dimethylaminomethyl, Dimethylaminoethyl, Methylaminomethyl, Methylaminoethyl oder Benzyl steht.

2. Schädlingsbekämpfungsmittel, **gekennzeichnet durch** einen Gehalt an mindestens einer Verbindung der Formel (I) nach Anspruch 1.

3. Verfahren zur Bekämpfung von Schädlingen, **dadurch gekennzeichnet, dass** man Verbindungen der Formel (I) nach Anspruch 1 auf Schädlinge und/oder ihren Lebensraum einwirken lässt.

4. Verwendung von Verbindungen der Formel (I) nach Anspruch 1 zur Bekämpfung von Schädlingen.

5. Verfahren zur Herstellung von Schädlingsbekämpfungsmitteln, **dadurch gekennzeichnet, dass** man Verbindungen der Formel (I) nach Anspruch 1 mit Streckmitteln und/oder oberflächenaktiven Mitteln vermischt.

6. Verfahren zur Herstellung von Verbindungen der Formel (I), gemäß Anspruch 1, **dadurch gekennzeichnet, dass** man
a) Hydroxyverbindungen der allgemeinen Formel (II) 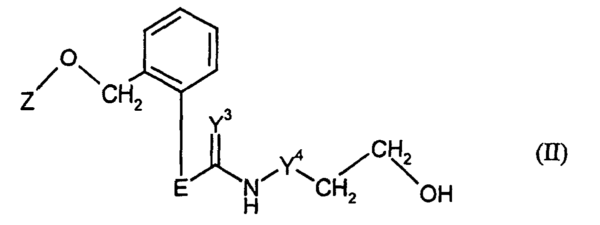 in welcher
E und Z die in Anspruch 1 angegebene Bedeutung haben, wobei
Y³ für eine Imino-Gruppierung ("Azamethylen", NH) steht und
Y⁴ für Sauerstoff steht,
mit einem Kondensationsmittel, gegebenenfalls in Gegenwart eines Verdünnungsmittels, umsetzt,
oder wenn man
b) Stickstoffhaltige Carbonsäurederivate der allgemeinen Formel (III) 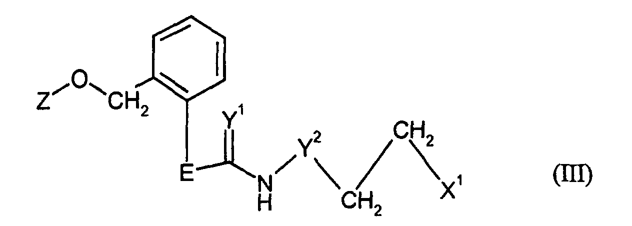 in welcher
Y¹ für eine Imino-Gruppierung ("Azamethylen", NH) steht,
Y² für Sauerstoff steht,
E und Z die in Anspruch 1 angegebene Bedeutung haben und
X¹ für Halogen, Arylsulfonyl oder Alkylsulfonyl steht,
mit einem Säureakzeptor, gegebenenfalls in Gegenwart eines Verdünnungsmittels, umsetzt.

## Claims

1. Compounds of the general formula (I) 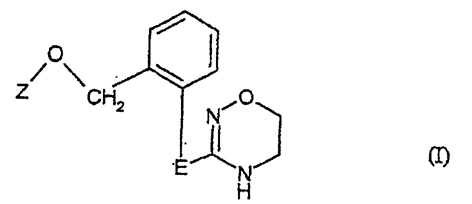 in which
E represents one of the following groups: 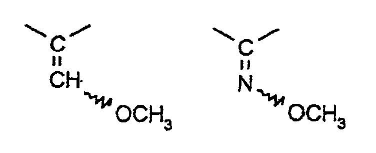 and
Z represents phenyl in each case optionally substituted by from one to three identical or different substituents, the possible substituents preferably being selected from the following list: fluorine, chlorine, bromine, cyano, methyl, ethyl, n- or i-propyl, n-, i-, s- or t-butyl, methoxy, ethoxy, n- or i-propoxy, methylthio, ethylthio, nor i-propylthio, methylsulphinyl, ethylsulphinyl, methylsulphonyl or ethylsulphonyl, trifluoromethyl, difluoromethoxy, trifluoromethoxy, difluorochloromethoxy, trifluoroethoxy, difluoromethylthio, trifluoromethylthio, difluorochloromethylthio, trifluoromethylsulphinyl or trifluoromethylsulphonyl, methoxycarbonyl, ethoxycarbonyl, in each case divalent methylenedioxy or ethylenedioxy in each case optionally substituted by from one to four identical or different substituents consisting of fluorine, chlorine, methyl, trifluoromethyl or ethyl,
or a group 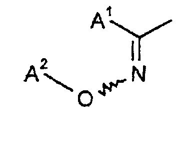 in which
A¹ represents methyl, ethyl, n- or i-propyl, n-, i-, s- or t-butyl, cyclopropyl or cyclobutyl and
A² represents methyl, ethyl, n- or i-propyl, n-, i-, s- or t-butyl, allyl, propargyl, but-2-en-1-yl, 2-methyl-prop-1-en-3-yl, cyanomethyl, methoxymethyl, ethoxymethyl, methoxyethyl, ethoxyethyl, methylthiomethyl, ethylthiomethyl, methylthioethyl, ethylthioethyl, dimethylaminoethyl, dimethylaminoethyl, methylaminomethyl, methylaminoethyl or benzyl.

2. Pest control composition **characterized by** the presence therein of at least one compound of the formula (I) according to Claim 1.

3. Method of controlling pests, **characterized in that** compounds of the formula (I) according to Claim 1 are caused to act on pests and/or their habitat.

4. Use of compounds of the formula (I) according to Claim 1 for controlling pests.

5. Process for preparing pest control compositions, **characterized in that** compounds of the formula (I) according to Claim 1 are mixed with extenders and/or surface-active agents.

6. Process for the preparation of compounds of the formula (I), according to Claim 1, **characterized in that**
a) hydroxyl compounds of the general formula (II) 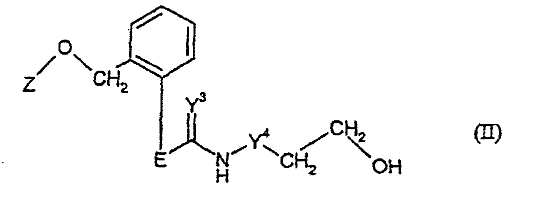 in which
E and Z have the meaning given in Claim 1 and where
Y³ represents an imino grouping ("azamethylene", NH) and
Y⁴ represents oxygen,
are reacted with a condensing agent, optionally in the presence of a diluent,
or if
b) nitrogen-containing carboxylic acid derivatives of the general formula (III) 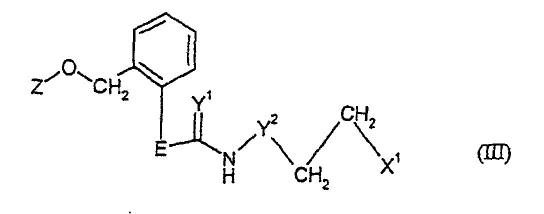 in which
Y¹ represents an imino grouping ("azamethylene", NH),
Y² represents oxygen,
E and Z have the meaning given in Claim 1 and
X¹ represents halogen, arylsulphonyl or alkylsulphonyl
are reacted with an acid acceptor, optionally in the presence of a diluent.

## Revendications

1. Composés de formule générale (I) 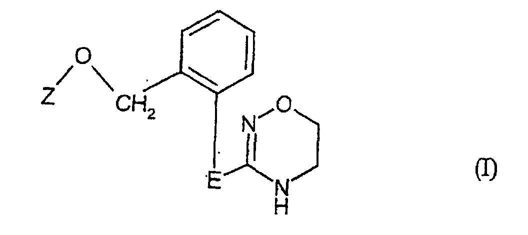 dans laquelle
E représente l'un des groupements suivants 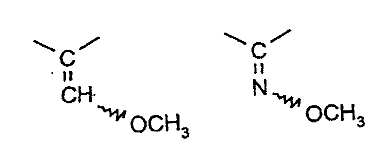 et
Z est un reste phényle portant éventuellement un à trois substituants identiques ou différents, les substituants possibles étant choisis avantageusement parmi ceux qui sont énumérés ci-après :
fluor, chlore, brome, cyano, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, difluoror:éthoxy, trifluorométhoxy, difluorochlorométhoxy, trifluoréthoxy, difluorométhylthio, trifluorométhylthio, difluorochlorométhylthio, trifluorométhylsulfinyle, trifluorométhylsulfonyle, méthoxycarbonyle, éthoxycarbonyle, méthylènedioxy ou éthylènedioxy ayant chacun deux liaisons et portant chacun, le cas échéant, un à quatre substituants fluoro, chloro, méthyle, trifluorométhyle ou éthyle identiques ou différents,
ou un groupement 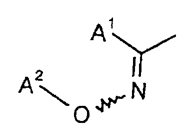 dans lequel
A¹ est un reste méthyle, éthyle, n-propyle, isopropyle, n-butyle, isobutyle, sec.-butyle, tertio-butyle, cyclo-propyle ou cyclobutyle et
A² est un reste méthyle, éthyle, n-propyle, isopropyle, n-butyle, isobutyle, sec.-butyle, terio-butyle, allyle, propargyle, but-2-ène-1-yle, 2-méthylprop-1-ène-3-yle, cyanométhyle, méthoxyméthyle, éthoxyméthyle, méthoxyéthyle, éthoxyéthyle, méthylthiométhyle, éthylthiométhyle, méthylthio-éthyle, éthylthio-éthyle, diméthylaminométhyle, diméthylamino-éthyle, méthylaminométhyle, méthylamino-éthyle ou benzyle.

2. Composition pesticide, **caractérisée par** une teneur en au moins un composé de formule (I) suivant la revendication 1.

3. Procédé pour combattre des parasites, **caractérisé en ce qu'**on fait agir des composés de formule (I) suivant la revendication 1 sur les parasites et/ou sur leur milieu.

4. Utilisation de composés de formule (I) suivant la revendication 1, pour combattre des parasites.

5. Procédé de préparation de compositions pesticides, **caractérisé en ce qu'**on mélange des composés de formule (I) suivant la revendication 1, avec des diluants et/ou des agents tensio-actifs.

6. Procédé de production de composés de formule (I) suivant la revendication 1, **caractérisé en ce que** :
(a) on fait réagir des composés hydroxylés de formule générale (II) 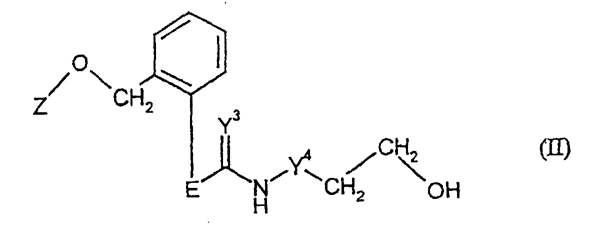 dans laquelle
E et Z ont la définition indiquée dans la revendication 1,
Y³ est un groupement imino ("azaméthylène", NH) et
Y⁴ représente l'oxygène,
avec un agent de condensation, éventuellement en présence d'un diluant,
ou **en ce que** :
b) on fait réagir des dérivés d'acides carboxyliques contenant de l'azote de formule générale (III) 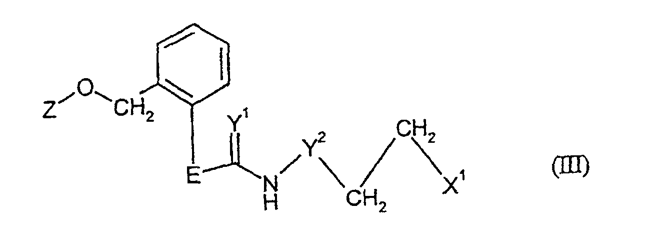 dans laquelle
Y¹ est un groupement imino ("azaméthylène", NH),
Y² est l'oxygène,
E et Z ont la définition indiquée dans la revendication 1 et
X¹ est un halogène, un reste arylsulfonyle ou alkylsulfonyle,
avec un accepteur d'acide, éventuellement en présence d'un diluant.
